# EUROPEAN PATENT APPLICATION

(11) **EP 2 884 497 A1**
(43) Date of publication of application: **17.06.2015**
(21) Application number: 13879630.5
(22) Date of filing: 08.08.2013
(51) Int. Cl.: G21C 17/02, G01N 23/00, G01N 27/64, G21C 17/00, G21D 3/08, G21F 9/02

(54) **HYDROGEN CONCENTRATION METER**

(30) Priority: 11.08.2012 JP 2012179127
(71) Applicant: YAMAMOTO, Kazuhiro, Bunkyo-ku, Tokyo 112-0002 (JP)
(72) Inventor: KATO, Kazuaki, Tsukuba-shi Ibaraki 300-4352 (JP); YAMAMOTO, Kazuhiro, Tokyo 112-0002 (JP)
(74) Representative: Hoefer & Partner
(86) International application number: PCT/JP2013/004805
(87) International publication number: WO 2014/027454

(57) **Abstract**

Apparatus (hydrogen concentration meter) is provided which can measure the density of hydrogen in monitored gas even in environments with high radiation dose, requires no driving electric source, and can measure the density of hydrogen in monitored gas even when the temperature surrounding apparatus becomes high. Hydrogen concentration meter 100 related to the present invention is apparatus for measuring the density of hydrogen in gas, comprising: a first electrode 1 formed of a first metal; a second electrode 2 facing the first electrode 1 and formed of a second metal having work function different from that of the first metal; and at least one of the first electrode 1 and the second electrode 2 detecting electrically charged particle generated electrically between the first electrode 1 and the second electrode 2 by recoil proton generated by irradiated neutron.

## Description

### Technical Field

The present invention relates to apparatus of measuring hydrogen density (hereafter referred to as hydrogen concentration meter), particularly to hydrogen concentration meter usable even in harsh environment.

### Description of the Related Art

### Background Art

As disclosed in Patent Document 1, for example, there exists a method for measuring the density of hydrogen in monitored gas inside of a nuclear reactor, namely in a harsh environment.

### Citation List

### Patent Document

PTL: Japanese Patent Application No. H6-130177

### Summary of Invention

### Problems to be solved by the Invention

There are some hydrogen concentration meters which are intended to be used in a harsh environment, such as an inside of a nuclear reactor, and which are provided with a unit for measuring the density of hydrogen and an electric source driving the unit. For hydrogen concentration meters of this kind, there are situations where they cannot measure the density of hydrogen when the electric source driving the unit is lost.

In addition, some of the above mentioned hydrogen concentration meters are provided internally with plastic components, for instance. The heat resistant temperature of plastic materials is approximately from 60 degrees centigrade to 100 degrees centigrade for general purpose plastic materials used widely (as an example, PMMA; Polymethyl Methacrylate). Therefore, there are situations where hydrogen concentration meters provided internally with components made of general purpose plastic cannot measure the density of hydrogen when the ambient temperature of the hydrogen concentration meters becomes high (500 degrees centigrade, for instance) and the plastic materials become softened and melted.

As described above, there are situations where hydrogen concentration meters related to conventional art cannot measure the density of hydrogen in monitored gas in harsh environments.

The present invention is invented with the above mentioned situations in mind, and the obj ect of the present invention is to provide hydrogen concentration meter which can measure the density of hydrogen in the monitored gas in an environment with high radiation dose, is not provided with any driving electric source, and can measure the density of hydrogen even when the ambient temperature of the hydrogen concentration meter becomes high.

### Solution to the Problem

In order to resolve the above problems, a hydrogen concentration meter related to an embodiment of the present invention is comprising: a first electrode being formed of a first metal; a second electrode being formed of a second metal having work function being different from that of said first metal, the second electrode facing said first electrode; and one of said first and second electrodes detecting electrically charged particle generated electrically between saidfirst and second electrodes by recoil proton generated by irradiated neutron.

This hydrogen concentration meter measures the density of hydrogen by deriving the density from the amount of charged particle (electric charge) ionizedby recoil proton. Inotherwords, thishydrogen concentration meter measures the density of hydrogen by utilizing ionization power (capability of ionizing gas) of recoil proton. Therefore, it can measure the density of hydrogen in monitored gas in an environment with high radiation dose.

In addition, this hydrogen concentration meter measures the amount of charged particle by utilizing contact potential difference occurring between the first and second electrodes. Therefore, this hydrogen concentration meter can be configured to measure the density of hydrogen in monitored gas without utilizing any driving electric source.

In addition, main components of this hydrogen concentration meter are pairs of electrodes made of metal, and this hydrogen concentration meter can be easily configured without components made of materials having necessarily the low softening/melting point (such as materials having the melting point of less than 500 degrees centigrade). With the aforementioned configuration, this hydrogen concentration meter can measure the density of hydrogen even when the ambient temperature of the hydrogen concentration meter becomes high (for instance, greater than or equal to 500 degrees centigrade, or greater than or equal to 1000 degrees centigrade depending upon metal used as electrodes).

In addition, the above mentioned hydrogen concentration meter can be provided with neutron source being arranged between said first and second electrodes, said neutron passing between said first and second electrodes in extending direction of said first and second electrodes.

This hydrogen concentration meter has a neutron source so that the neutron may pass between the first and second electrodes in extending direction of the first and second electrodes. With the neutron source, the amount of neutron passing between the first and second electrodes increases compared with a hydrogen concentration meter without the neutron source. Therefore, this hydrogen concentration meter can measure the density of hydrogen in higher sensitivity.

In addition, the above mentioned hydrogen concentration meters maybe provided with voltage applied to said first and second electrodes. This hydrogen concentration meter, with voltage applied to the first and second electrodes, can capture charged particle, ionized by recoil proton, with the first electrode or the second electrode more efficiently than a hydrogen concentration meter without the voltage applied between the electrodes. Therefore, this hydrogen concentration meter can measure the density of hydrogen in higher sensitivity.

### Effects of the Invention

Hydrogen concentration meter related to the present invention measures the density of hydrogen by deriving the density from the amount of charged particle (electric charge) ionized by recoil proton. Therefore, it can measure the density of hydrogen in the monitored gas in an environment with high radiation dose rate.

In addition, this hydrogen concentration meter measures the amount of charged particle by utilizing contact potential difference occurring between the first and second electrodes. Therefore, this hydrogen concentration meter can be configured to measure the density of hydrogen in the monitored gas without utilizing any driving electric source.

In addition, main components of this hydrogen concentration meter are pairs of electrodes made of metal, and this hydrogen concentration meter can be easily configured without components made of materials having necessarily the low softening/melting point. With the aforementioned configuration, this hydrogen concentration meter can measure the density of hydrogen even when the ambient temperature of the hydrogen concentration meter becomes high.

### Brief Description of the Drawings

Fig. 1 schematically illustrates a structure of a hydrogen concentration meter according to the first embodiment of the present invention.
Fig. 2 schematically illustrates a structure of a hydrogen concentration meter according to the second embodiment of the present invention.
Fig. 3 schematically illustrates a structure of a hydrogen concentration meter according to the third embodiment of the present invention.
Fig. 4 schematically illustrates a structure of a hydrogen concentration meter according to the fourth embodiment of the present invention.
Fig. 5 schematically illustrates a structure of a hydrogen concentration meter according to the fifth embodiment of the present invention.

### Description of Emobodiments

### <First Embodiment>

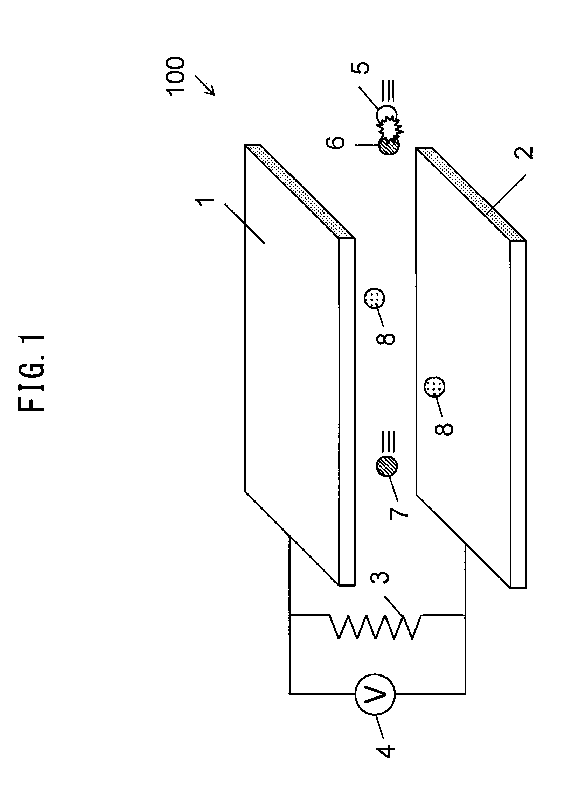

Fig. 1 schematically illustrates a structure of a hydrogen concentration meter according to the first embodiment of the present invention. Hereinafter, while referring to Fig. 1, the structure, operation and effect of a hydrogen concentration meter related to the present embodiment is explained.

### <Structure>

A hydrogen concentration meter 100 related to the present embodiment is provided with a first electrode 1 and a second electrode 2 arranged to face the first electrode 1. In addition, the first electrode 1 and the secondelectrode 2 are electrically connected through a resistor 3. In addition, the hydrogen concentration meter 100 is provided with a voltmeter 4 which is connected in parallel with the resister 3.

An example of the first electrode 1 and the second electrode 2 is a planar electrode, and there is an empty space between the first electrode 1 and the second electrode 2, which are arranged to face each other. The space between the first electrode 1 and the second electrode 2 is an open space. In addition, in the space, no object is installed which disturbs the flow of air (monitored gas) around the hydrogen concentration meter 100, and the air can flow freely between the first electrode 1 and the second electrode 2.

The first electrode 1 is formed of a first metal, and the second electrode 2 is formed of a second metal whose work function is different from that of the first metal. Here, although the first metal and the second metal are preferably selected so that the difference (contact potential difference) between their work functions may be large, any pair of metals may be used as long as their work functions differ.

The first metal forming the first electrode 1 may, for example, be one of: Li, Be, Na, Mg, Al, K, Ca, Sc, Ti, V, Cr, Mn, Fe, Co, Ni, Cu, Zn, Ga, Ge, Rb, Sr, Y, Zr, Nb, Mo, Tc, Ru, Rh, Pd, Ag, Cd, IN, Sn, Sb, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Po, Fr, Ra, Ac, Th, Pa, U, Np, Pu, Am, Cm, Bk, Cf, Es, Fm, Md, No, Lr, Rf, Db, Sg, Bh, Hs, Mt, Ds; or an alloy formed by mixing several metals selected from the above metals.

The second metal forming the second electrode 1 may, for example, be one of: Li, Be, Na, Mg, Al, K, Ca, Sc, Ti, V, Cr, Mn, Fe, Co, Ni, Cu, Zn, Ga, Ge, Rb, Sr, Y, Zr, Nb, Mo, Tc, Ru, Rh, Pd, Ag, Cd, IN, Sn, Sb, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Po, Fr, Ra, Ac, Th, Pa, U, Np, Pu, Am, Cm, Bk, Cf, Es, Fm, Md, No, Lr, Rf, Db, Sg, Bh, Hs, Mt, Ds; or an alloy formed by mixing several metals selected from the above metals.

The resistor 3 and the voltmeter 4 can be provided, respectively, with a well-known resistor and a well-known voltmeter as examples. Therefore, their explanations are omitted here.

### <Operation>

Hereinafter, a method of measuring the density of hydrogen by using the hydrogen concentration meter 100 related to the present embodiment is explained.

The hydrogen concentration meter 100 related to the present embodiment determines the density of hydrogen in monitored gas by utilizing ionization power of a recoil proton 7 generated by elastic scattering of a neutron 5 and a proton (hydrogen atomic nuclei) 6. Therefore, it is assumed that the hydrogen concentration meter 100 is used in an environment where a certain amount of neutron 5 exist around the hydrogen concentration meter 100, such as inside of a nuclear reactor, in places with high radiation dose, and in places where a neutron source is available.

Firstly, it is supposed that a certain amount of hydrogen exists in the monitored gas existing between the first electrode 1 and the second electrode 2 arranged to face each other. A proton 6 that forms hydrogen is caused to become a recoil proton 7 by a neutron 5 emitted fromaneutronsource (notillustratedinthefigure), such as radioactive isotope including Cf-252 and Am-242+Be. If the amount of neutron 5 irradiated to proton 6 is assumed to be fixed (or, normalized), the amount of recoil proton 7 is directly proportional to the amount of hydrogen existing between the first electrode 1 and the second electrode 2, namely, the density of hydrogen in the monitored gas.

When the above recoil proton 7 is generated, the proton 6 receives kinetic energy from the neutron 5 with high efficiency. This is because the mass of the proton 6 is smaller than that of other atomic nucleus, such as that of oxygen and nitrogen. The recoil proton 7 having received kinetic energy from the neutron 5 with high efficiency can easily ionize the monitored gas (regardless of kinds of molecules and atoms forming the monitored gas) existing between the first electrode 1 and the second electrode 2, and generates charged particle 8. Because the amount of generated recoil proton 7 is directly proportional to the density of hydrogen in the monitored gas as described above, the amount of the generated charged particle 8 is directly proportional to the density of hydrogen in the monitored gas.

The present embodiment detects generated charged particle 8 by utilizing contact potential difference occurring between the first electrode 1 and the second electrode 2. As mentioned above, the first electrode 1 and the second electrode 2 are individually formed of metals with different work functions. If metals of two kinds having work functions different from each other are electrically connected through a metal wire, for instance, contact potential difference occurs between the first electrode 1 and the second electrode 2. By utilizing the potential difference, the charged particle 8 generated by recoil proton 7 can be detected with either the first electrode 1 or the second electrode 2. In addition, the amount of generated charged particle 8 (namely, amount of electric charge) is measured as electromotive force generated between the first electrode 1 and the secondelectrode 2 with the voltmeter 4 .

By comparing the voltage actually measured (the quantity correlated with ionization power of recoil proton 7), as mentioned above, with voltage values measured and recorded in a conversion table in advance, the density of hydrogen in monitored gas (the density of hydrogen between the first electrode 1 and the second electrode 2) can be determined.

It should be noted that although the present embodiment is explained with a case where the first electrode 1 and the second electrode 2 are planar electrodes, the embodiment is not limited to the case. The shapes of the electrode 1 and the electrode 2 can be adjusted according to the shape of the place where the hydrogen concentration meter 100 is installed. For instance, when the hydrogen concentration meter 100 is installed in a ceiling of a building, the shapes of the electrode 1 and the electrode 2 can be changed according to the shape of the ceiling of the building, for example. In addition, when the hydrogen concentration meter 100 is installed inside of a nuclear reactor, the shapes of the electrode 1 and the electrode 2 can be changed according to the shape of the nuclear reactor.

In addition, although the present embodiment is explained with a case where the first electrode 1 and the second electrode 2 are formed of metals or alloys, the embodiment is not limited to the case. The first electrode 1 and the second electrode 2 can be formed of inorganic material having electroconductivity or organic material having lectroconductivity, instead of metals or alloys as mentioned above. For instance, inorganic material having electroconductivity includes concrete having electroconductivity. In addition, organic material having electroconductivity includes resign material having electroconductivity, for instance.

### <Effect>

As described above, the hydrogen concentration meter 100 related to the present embodiment measures the density of hydrogen by deriving the density of hydrogen from the amount of charged particle 8 generated by recoil proton 7 and detected with the first electrode 1 or the second electrode 2. In other words, the hydrogen concentration meter 100 measures the density of hydrogen by utilizing ionization power (capability of ionizing gas) of recoil proton 7. Therefore, it can measure the density of hydrogen in monitored gas in an environment with high radiation dose.

In addition, the hydrogen concentration meter 100 measures the amount of charged particle 8 (amount of electric charge) generated by recoil proton 7 by utilizing contact potential difference occurring between the first electrode 1 and the second electrode 2. Therefore, the hydrogen concentration meter 100 can be configured to measure the density of hydrogen in the monitored gas without utilizing any driving electric source.

In addition, main components of the hydrogen concentration meter 100 are pairs of electrodes 1 and 2 made of metal, and the hydrogen concentration meter 100 can be easily configured without components made of materials having necessarily the low softening/melting point. Because of that, the hydrogen concentration meter 100 can measure the density of hydrogen even when the ambient temperature of the hydrogen concentration meter 100 becomes high (for instance, greater than or equal to 500 degrees centigrade, or greater than or equal to 1000 degrees centigrade depending upon metal used as electrodes).

In this way or manner, the hydrogen concentration meter 100 related to the present embodiment can measure the density of hydrogen in monitored gas even in harsh environment (for example, inside of a nuclear reactor) and place (such as one with high radiation dose rate) where a person can not enter. It should be noted that when the hydrogen concentration meter 100 related to the present embodiment is installed in a building for a nuclear reactor, for example, it is preferable that the hydrogen concentration meter 100 is installed inside the building and near the ceiling of the building.

### <Second Embodiment>

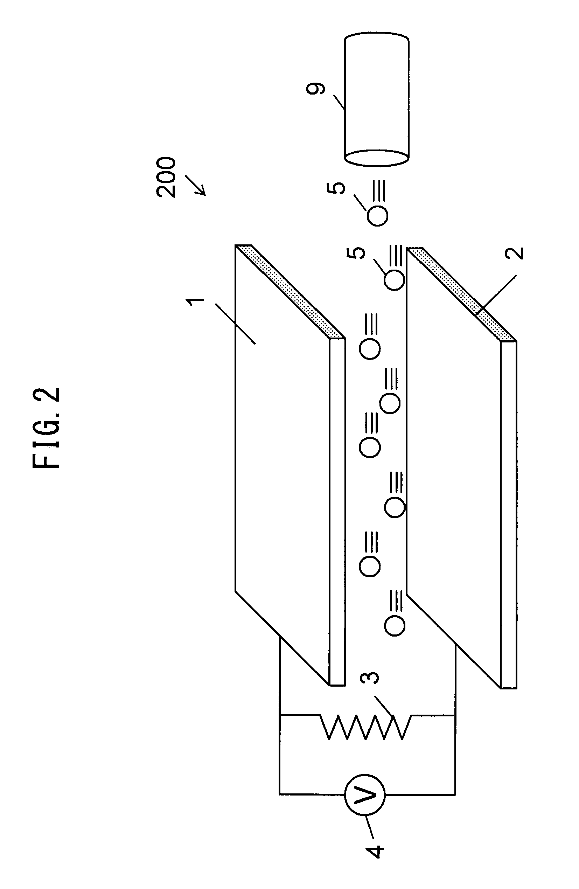

Fig. 2 schematically illustrates a structure of a hydrogen concentration meter 200 according to the second embodiment of the present invention. As Fig. 2 illustrates, the hydrogen concentration meter 200 related to the present embodiment has nearly the same structure as that of the hydrogen concentration meter 100 related to the first embodiment. However, the hydrogen concentration meter 200 differs in its structure from the hydrogen concentration meter 100 in that it is provided with a neutron source 200 so that neutron 5 may pass between the first electrode 1 and the second electrode 2. Therefore, for the present embodiment, only this different part is explained and the remaining parts are not referred to. It should be noted that, in Fig. 2, the same numbers are assigned to the same components as components explained in Fig. 1. In addition, although Fig. 2 omits illustrations of recoil proton 7 and charged particle 8, it is assumed, similarly to the first embodiment, that recoil proton 7 is generated by neutron 5, and charged particle 8 is generated by the recoil proton 7 in the present embodiment.

The neutron source 9 is arranged so that the neutron 5 may pass between the first electrode 1 and the second electrode 2 in extending direction of the first electrode 1 and the second electrode 2. It should be noted that the installation position of the neutron source 9 is irrelevant as long as the neutron 5 may pass between the first electrode 1 and the second electrode 2 in extending direction of the first electrode 1 and the second electrode 2. In addition, examples of the above neutron source 9 include radioactive isotope, such as Cf-252 and Am-242+Be (whose energy is approximately several MeV at most), as well as a proton accelerator (linear accelerator as an example) generating neutron (whose energy is approximately 14MeV) by utilizing nuclear reaction D (p, n). It should be noted that when radioactive isotope is utilized as the neutron source 9, the radioactive isotope may be embedded in parts of electrodes 1 and 2, or may be coated on parts of the surface of the electrodes. In addition, the radioactive isotope may be coated on the entire surface of the electrodes 1 and 2 if electric conductivity of the electrodes is not decreased.

As described above, the hydrogen concentration meter 200 related to the present embodiment can increase the amount of neutron 5 passing between the first electrode 1 and the second electrode 2 in extending direction of the first electrode 1 and the second electrode 2 compared to a hydrogen concentration meter without neutron source. Therefore, the amount of recoil proton 7 between the first electrode 1 and the second electrode 2 can be increased, and the amount of charged particle 8 can be increased. Consequently, the hydrogen concentration meter 200 can measure the density of hydrogen in monitored gas in higher sensitivity.

### <Third Embodiment>

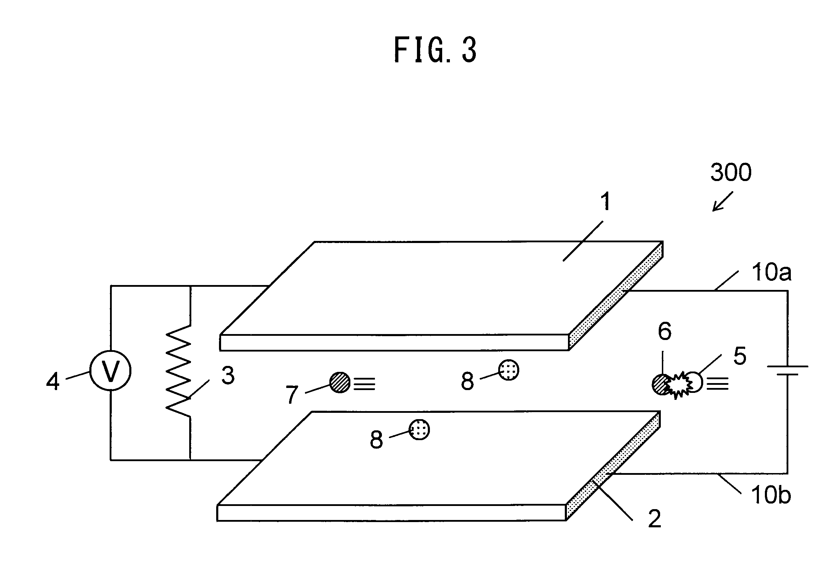

Fig. 3 schematically illustrates a structure of a hydrogen concentration meter 300 according to the third embodiment of the present invention. As Fig. 3 illustrates, the hydrogen concentration meter 300 related to the present embodiment has nearly the same structure as that of the hydrogen concentration meter 100 related to the first embodiment. However, the hydrogen concentration meter 300 differs in its structure from the hydrogen concentration meter 100 in that voltage is separately applied to the first electrode 1 and the second electrode 2. Therefore, for the present embodiment, only this different part is explained and the remaining parts are not referred to. It should be noted that, in Fig. 3, the same numbers are assigned to the same components as components explained in Fig. 1.

A metal wiring 10a is connected to the first electrode 1, and there exists a structure so that voltage may be applied to the first electrode 1 through the metal wiring 10a. In addition, as with the first electrode 1, a metal wiring 10b is connected to the second electrode 2, and there exists a structure so that voltage may be applied to the second electrode 2 through the metal wiring 10b.

The metal wiring 10a connected to the first electrode 1 may be made of the same metal as the metal wiring 10b connected to the second electrode 2, or a different metal from the metal wiring 10b.

Although Fig. 3 illustrates that positive potential is applied to the first electrode 1 and negative potential is applied to the second electrode 2, polarities of potentials for electrodes may be exchanged. This means that negative potential can be applied to the first electrode 1 and positive potential can be applied to the second electrode 2. It should be noted that the above mentioned voltage applied to the electrodes 1 and 2 may be chosen arbitrarily if stability of output with respect to variation of applied voltage is considered unimportant, but, otherwise, the voltage should be preferably set in a "saturation range" in V-I characteristics, and the range can be obtained easily experimentally. The voltage is usually set in the range between several volts to several tens of volts, but, it does not need to be.

As described above, the hydrogen concentration meter 300 related to the present embodiment, with voltage applied to the first electrode 1 and the second electrode 2, can capture charged particle 8, ionized by recoil proton 7, with the first electrode 1 or the second electrode 2 more efficiently than a hydrogen concentration meter without the voltage applied to the first electrode 1 and the second electrode 2. Therefore, this hydrogen concentration meter can measure the density of hydrogen in higher sensitivity.

### <Fourth Embodiment>

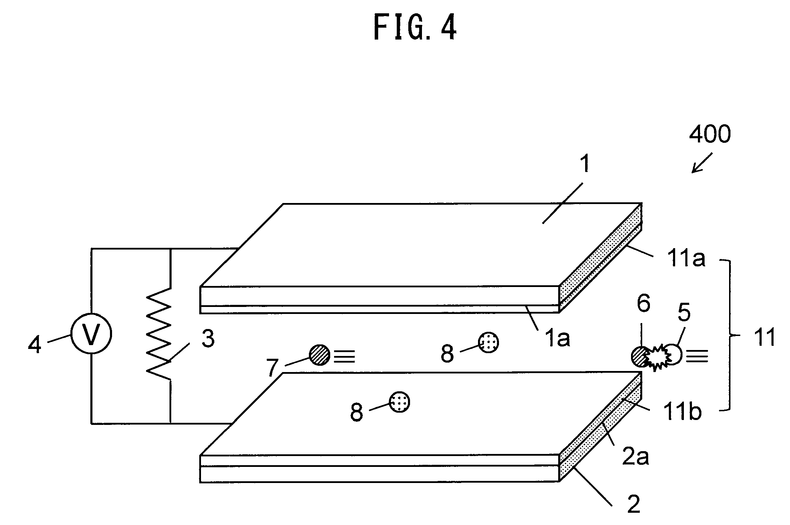

Fig. 4 schematically illustrates a structure of a hydrogen concentration meter 400 according to the fourth embodiment of the present invention. As Fig. 4 illustrates, the hydrogen concentration meter 400 related to the present embodiment has nearly the same structure as that of the hydrogen concentration meter 100 related to the first embodiment. However, the hydrogen concentration meter 400 differs in its structure from the hydrogen concentration meter 100 in that surfaces of the first electrode 1 and the second electrode 2 are covered with metal thin films 11. Therefore, for the present embodiment, only this different part is explained and the remaining parts are not referred to. It should be noted that, in Fig. 4, the same numbers are assigned to the same components as components explained in Fig. 1.

The surface 1a, of the first electrode 1, facing the second electrode 2 is covered with the first metal thin film 11a. In addition, the surface 2a, of the second electrode 2, facing the first electrode 1 is covered with the second metal thin film 11b. Here, although the metal used for the first metal thin film 11a and the metal used for the secondmetal thin film 11b are preferably selected so that difference (contact potential difference) between their work functions may be large, any pair of metals may be used as long as their work functions differ.

The first metal thin film may, for example, be made of one of: Li, Be,Na, Mg, Al, K, Ca, Sc, Ti, V, Cr, Mn, Fe, Co, Ni, Cu, Zn, Ga, Ge, Rb, Sr, Y, Zr,Nb, Mo, Tc, Ru, Rh, Pd, Ag, Cd, IN, Sn, Sb, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Po, Fr, Ra, Ac, Th, Pa, U, Np, Pu, Am, Cm, Bk, Cf, Es, Fm, Md, No, Lr, Rf, Db, Sg, Bh, Hs, Mt, Ds; or an alloy formed by mixing several metals selected from the above metals.

In addition, the second metal thin film may, for example, be made of one of: Li, Be, Na, Mg, Al, K, Ca, Sc, Ti, V, Cr, Mn, Fe, Co, Ni, Cu, Zn, Ga, Ge, Rb, Sr, Y, Zr, Nb, Mo, Tc, Ru, Rh, Pd, Ag, Cd, IN, Sn, Sb, Cs, Ba, La, Ce, Pr, Nd, Pro, Sra, Eu, Gd, Tb, Dy, Ho, Er, Tm, Yb, Lu, Hf, Ta, W, Re, Os, Ir, Pt, Au, Hg, Tl, Pb, Bi, Po, Fr, Ra, Ac, Th, Pa, U, Np, Pu, Am, Cm, Bk, Cf, Es, Fm, Md, No, Lr, Rf, Db, Sg, Bh, Hs, Mt, Ds; or an alloy formed by mixing several metals selected from the above metals.

The present embodiment can also cause contact potential difference to occur between the first electrode 1 (the first metal thin film 11a) and the second electrode 2 (the second metal thin film 11b). Therefore, the present embodiment has the same effects as the first embodiment.

### <Fifth Embodiment>

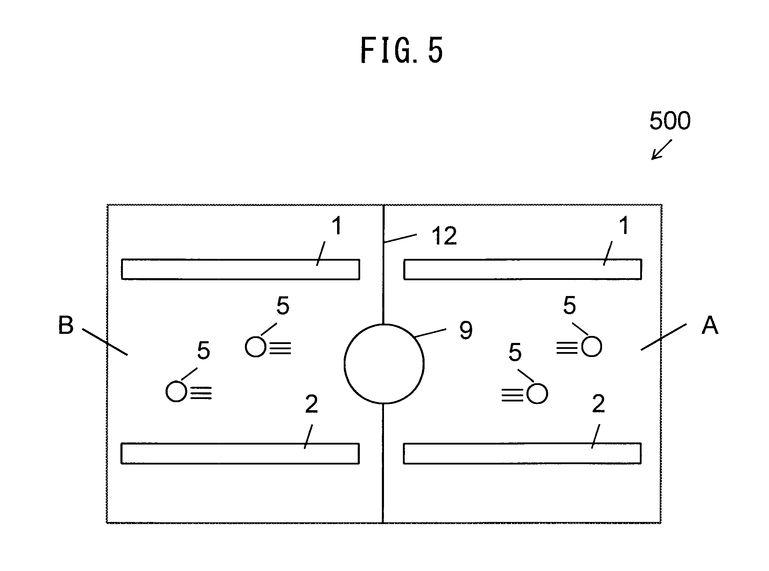

Fig. 5 schematically illustrates a structure of a hydrogen concentration meter 500 according to the fifth embodiment of the present invention. As Fig. 5 illustrates, the hydrogen concentration meter 500 related to the present embodiment is provided with two adjacent containers A and B. And, hydrogen concentration meters 100 are respectively arranged in the containers A and B. Therefore, for the present embodiment, only this different part is explained and the remaining parts are not referred to. It should be noted that, in Fig. 5, the same numbers are assigned to the same components as components explained in Fig. 1.

The hydrogen concentrationmeter 500 is providedwith two adj acent containers A and B, as Fig 5 illustrates. The inner space of the container A in the right side in the figure is a closed space. The closed space is filled completely with only nitrogen and oxygen, and does not contain hydrogen. On the contrary, the inner space of the container B in the left side of the figure is an open space. For example, the container B is provided with openings (not illustrated in the figure) on both front and back sides, and monitored gas, such as one containing hydrogen, may pass through the container B from the front side to the back side.

Pairs of electrode 1 and electrode 2 are respectively arranged in the containers A and B just like the hydrogen concentration meter 100. In addition, a neutron source 9 is arranged in a partition wall 12 which separates the containers A and B. Furthermore, the neutron source 9 emits the same amount of neutron into the containers A and B. Since amechanism whereby the emitted neutron generates recoil proton 7 and the recoil proton 7 generates charged particle 8 is explained in the first embodiment, its explanation is omitted in the following description. In addition, Fig. 5 omits illustrations of recoil proton 7 and charged particle 8.

The hydrogen concentration meter 500 related to the present embodiment can utilize the electromotive force generated between a pair of electrodes 1 and 2 arranged in the container A as a reference of electromotive force (hereinafter referred to as "reference electromotive force"). By comparing the reference electromotive force and an electromotive force generated between a pair of electrodes 1 and 2 arranged in the container B, the density of hydrogen in monitored gas can be measured more accurately.

It should be noted that the present embodiment is explained with an arrangement where the same amount of neutron is emitted into the containers A andB, but the present embodiment is not limited thereto. For example, even when neutron 5 emitted toward the container A may be obstructed and neutron 5 is emitted only into the container B, the same effect as those with the above embodiment is achieved. It should be noted that when emitted neutron 5 is obstructed, members preventing permeation of neutron is used, for example.

### <Variations>

A hydrogen concentration meter related to a variation of the above mentioned embodiments is a hydrogen concentration meter (not illustrated in the figures) which combines a hydrogen concentration meter 100 related to the first embodiment and a hydrogen concentration meter 300 related to the third embodiment. The hydrogen concentration meter related to the present variation may be arranged near the ceiling of a building for a nuclear reactor as an example. The hydrogen concentration meter related to the present variation can measure the density of hydrogen in monitored gas with the hydrogen concentration meter 300, which has a relatively higher sensitivity, when radiation dose is low. In addition, the present hydrogen concentration meter can measure the density of hydrogen in monitored gas with the hydrogen concentration meter 100 even when the temperature and/or radiation dose in the environment around the present hydrogen concentration meter becomes high, and thus the hydrogen concentration meter 300 can not be utilized.

As described above, the hydrogen concentration meter related to the present variation can measure the density of hydrogen in both an ordinary measurement environment and a harshmeasurement environment.

The present invention may be embodied in other specific forms without departing from the spirit or essential characteristics thereof. The embodiments disclosed in this application are to be considered in all respects as illustrative and not restrictive, the scope of the invention being indicated by the appended claims rather than by the foregoing description, all changes that come within the meaning and range of equivalency of the claims are intended to be embraced therein.

### Reference Signs List

- 1: First electrode
- 2a: Surface of the first electrode
- 2: Second electrode
- 2a: Surface of the second electrode
- 3: Resistance
- 4: Voltmeter
- 5: Neutron
- 6: Proton
- 7: Recoil proton
- 8: Charged particle
- 9: Neutron source
- 10a: Metal wiring
- 10b: Metal wiring
- 11: Metal thin film
- 11a: First metal thin film
- 11b: Second metal thin film
- 12: Partition wall
- 100: Hydrogen concentration meter
- 200: Hydrogen concentration meter
- 300: Hydrogen concentration meter
- 400: Hydrogen concentration meter
- 500: Hydrogen concentration meter
- A: Container
- B: Container

## Claims

1. A hydrogen concentration meter for measuring density of hydrogen in gas, comprising:
a first electrode formed of a first metal;
a second electrode formed of a second metal having work function different from the work function of the first metal, the second electrode facing the first electrode; wherein,
at least one of the first electrode or the second electrode detect an electrically charged particle generated electrically between the first electrode and the second electrode by a recoil proton generated by an irradiated neutron.

2. A hydrogen concentration meter according to claim 1, further comprising a neutron source arranged between the first electrode and the second electrode, wherein,
the neutron passes between the first electrode and the second electrode in extending direction of the first electrode and the second electrode.

3. A hydrogen concentration meter according to claim 1 or claim 2, wherein, voltage is applied to the first electrode and the second electrode.
